# EUROPEAN PATENT APPLICATION

(11) **EP 4 555 994 A1**
(43) Date of publication of application: **21.05.2025**
(21) Application number: 24212431.1
(22) Date of filing: 12.11.2024
(51) Int. Cl.: A61K 8/19, A61K 8/64, A61K 8/9789, A61K 33/00, A61K 36/704, A61K 38/00, A61P 17/14, A61Q 7/00

(54) **COMPOSITION FOR PROMOTING HAIR GROWTH**

(30) Priority: 14.11.2023 TW 112143897
(71) Applicant: Pell Bio-Med Technology Co., Ltd., Taipei City (TW)
(72) Inventor: LIN, Chen-Lung, Taipei City (TW); CHIEN, Wen-Pin, Taipei City (TW); CHUANG, Hui-Ying, Taipei City (TW)
(74) Representative: Valenza, Silvia

(57) **Abstract**

Provided is a composition for promoting hair growth, which comprises a mixture comprising an extract of *Polygonum Multiflorum* Thunb and silver nanoparticles; and Copper Tripeptide-1, wherein the weight/volume ratio of the Copper Tripeptide-1 to the composition for promoting hair growth is from 0.1 to 0.2 mg/mL; wherein the mixture comprising the extract of *Polygonum Multiflorum* Thunb and silver nanoparticles comprises the silver nanoparticles, extract of *Polygonum Multiflorum* Thunb, a stabilizer, and a diluent. The present invention further provides a pharmaceutical composition for promoting hair growth comprising the composition and a pharmaceutically acceptable excipient, and the use thereof in for promoting hair growth. The composition for promoting hair growth of the present invention can increase hair growth and stimulate the growth of new hair follicles.

## Description

### 1. Field of the Invention

The present invention relates to a composition which can be used for promoting hair growth, a pharmaceutical composition for promoting hair growth comprising the composition, and a method for promoting hair growth by using the composition or the pharmaceutical composition.

### 2. Description of the Prior Arts

Alopecia areata is a skin disease causing hair loss on the scalp or elsewhere on the body. Alopecia areata usually starts with one or more round, small, smooth bald patches on the scalp and can progress to total scalp hair loss (alopecia totalis) or complete body hair loss (alopecia universalis).

It is generally believed that alopecia areata is caused by autoimmune system attacking the hair follicles; also, another observation has pointed out that a hormone called dihydroxytestosteron (DHT) naturally occurring in our bodies may cause the shortened growth phase of hair, make the grown hairs thin and invisible, thereby resulting in hereditary male-pattern hair loss. If developing continuously, it will cause the receding male hairlines gradually.

Alopecia areata may occur in humans among all genders and races and may begin in adolescence. From an epidemiological perspective, alopecia areata affects 1.7 percent (1.7%) of the population all over the world, among which there are 4.7 million or more people in the United States alone. This above-mentioned condition may have an impact on one's appearance, self-confidence, life quality, and work or studies.

According to U.S. Patent No. 9295694, it has been known that the composition of silver nanoparticles (AgNPs) and an extract of *Polygonum Multiflorum* Thunb can be used to improve alopecia areata. However, the pursuit of more excellent effects is still a goal of current research and development in this technical field of the present invention.

In order to achieve the above objective, the present invention provides a composition for promoting hair growth which comprises a mixture comprising an extract of *Polygonum Multiflorum* Thunb and silver nanoparticles; and Copper Tripeptide-1; wherein a weight/volume ratio of the Copper Tripeptide-1 to the composition for promoting hair growth is from 0.1 milligrams/milliliter (mg/mL) to 0.2 mg/mL; wherein the mixture comprising the extract of *Polygonum Multiflorum* Thunb and the silver nanoparticles comprises the silver nanoparticles, the extract of *Polygonum Multiflorum* Thunb, a stabilizer, and a diluent.

Although it is currently known that AgNPs and an extract of *Polygonum Multiflorum* Thunb can be used to improve alopecia areata, the present invention further combines the Copper Tripeptide-1 and controls the ratio of the mixture comprising the extract of *Polygonum Multiflorum* Thunb and the silver nanoparticles and Copper Tripeptide-1; therefore, an unexpected, more excellent effect of hair growth promotion can be brought out.

According to the present invention, Copper Tripeptide-1 has a complex structure with copper as the central atom which is surrounded by molecules of glycine-histidine-lysine (Gly-His-Lys, GHK) tripeptides. In one embodiment, Copper Peptide of CAS No. 49557-75-7 may be adopted. In another embodiment, Copper Peptide of CAS No. 89030-95-5 may be adopted.

Preferably, a weight/volume ratio of the AgNPs to the mixture comprising the extract of *Polygonum Multiflorum* Thunb and the AgNPs may be from 0.001 mg/mL to 0.1 mg/mL. More preferably, the weight/volume ratio of the AgNPs to the mixture comprising the extract of *Polygonum Multiflorum* Thunb and the AgNPs may be from 0.005 mg/mL to 0.02 mg/mL. Even more preferably, the weight/volume ratio of the AgNPs to the mixture comprising the extract of *Polygonum Multiflorum* Thunb and the AgNPs may be from 0.0077 mg/mL to 0.014 mg/mL.

Preferably, the particle size of the silver nanoparticles may be smaller than or equal to 50 nanometers (nm); more preferably, the particle size of the silver nanoparticles may be smaller than or equal to 20 nm; even more preferably, the particle size of the silver nanoparticles may be smaller than or equal to 10 nm. Preferably, the particle size of the silver nanoparticles may be larger than or equal to 1 nm. According to the present invention, the particle size refers to the diameter thereof. In some embodiments, the particle size of the silver nanoparticles refers to the average particle size of the silver nanoparticles.

Preferably, a weight/volume ratio of the extract of *Polygonum Multiflorum* Thunb to the mixture comprising the extract of *Polygonum Multiflorum* Thunb and the silver nanoparticles may be from 0.1 mg/mL to 10 mg/mL. Preferably, the weight/volume ratio of the extract of *Polygonum Multiflorum* Thunb to the mixture comprising the extract of *Polygonum Multiflorum* Thunb and the silver nanoparticles may be from 0.5 mg/mL to 5 mg/mL. More preferably, the weight/volume ratio of the extract of *Polygonum Multiflorum* Thunb to the mixture comprising the extract of *Polygonum Multiflorum* Thunb and the silver nanoparticles may be from 0.91 mg/mL to 1.82 mg/mL.

Preferably, the weight/volume ratio of the Copper Tripeptide-1 to the composition for promoting hair growth may be from 0.1 mg/mL to 0.18 mg/mL. Preferably, the weight/volume ratio of the Copper Tripeptide-1 to the composition for promoting hair growth may be from 0.1 mg/mL to 0.16 mg/mL.

Preferably, the extract of *Polygonum Multiflorum* Thunb may be an extract of the roots of *Polygonum Multiflorum* Thunb.

Preferably, the manufacturing method of the extract of the *Polygonum Multiflorum* Thunb (PMT) may comprise: (a) mixing a PMT and an alcohol solvent and undergoing an extraction for 24 hours to 72 hours at a temperature from 40°C to 60°C, and then separating to remove the post-extracted PMT so as to obtain a PMT extract liquid; (b) removing the solvent in the PMT extract liquid and drying, so as to obtain an extract of the PMT thereof.

Preferably, the PMT in step (a) may be dried PMT.

Preferably, the alcohol solvent may comprise methanol, ethanol or propanol. The concentration of the alcohol solvent may be from 30 volume percent (vt%) to 95 vt%; wherein the alcohol solvent may be an aqueous solution.

Preferably, a solid-to-solution ratio (w:v) of the PMT to the alcohol solvent may be from 1: 1 to 1:30. For example, when the solid-to-solution ratio (w:v) of the PMT to the alcohol solvent is 1:1, it means that 100 grams (g) of the PMT is mixed with 100 mL of the alcohol solvent. When the solid-to-solution ratio (w:v) of the PMT to the alcohol solvent is 1:30, it means that 100 g of the PMT is mixed with 3000 mL of the alcohol solvent.

According to the present invention, the extract of PMT used in examples may be purchased from GREENTECH BIOTECHNOLOGIES. The extraction method is as follows: 100 g of the dried roots of PMT are mashed and immersed in 1000 mL of 50 vt% ethanol aqueous solution. After extracting for 48 hours, the resulting PMT roots extract liquid is obtained by separation and filtration, and the resulting PMT roots extract liquid is subsequently concentrated under reduced pressure and freeze dried; finally, an amount of 10 g of the extract of the roots of PMT is obtained. Wherein, the extraction rate of the extract of the roots of PMT is 10%.

Preferably, the stabilizer may comprise a protein, a peptide, sodium borohydride or polyvinyl pyrrolidone (PVP). Wherein, the protein may be human serum proteins or transferrin. Preferably, the stabilizer may be the PVP which can effectively stabilize the silver nanoparticles. Preferably, based on the total volume of the composition for promoting hair growth, the concentration of the PVP may be from 0.1 mM to 1 mM. In one embodiment, the concentration of the PVP may be 0.57 mM. Preferably, the diluent may be selected from the group consisting of: water, ethanol, dimethyl sulfoxide (DMSO), acetonitrile (ACN), Hepes buffer, a phosphate buffer, Tris buffer, a citrate buffer, serum or any other kinds of physiologically relevant solvents or solutions and any combinations thereof.

In order to achieve the above-mentioned objective, the present invention further provides a use of a composition for promoting hair growth for the manufacture of a pharmaceutical composition for promoting hair growth, wherein the composition for promoting hair growth is as described above.

The present invention further provides a pharmaceutical composition for promoting hair growth, comprising: the composition for promoting hair growth as described above; and a pharmaceutically acceptable excipient. The pharmaceutical composition may be a medical product; the medical product may be a drug or a medical device. The medical product is intended for administration to a subject in need thereof.

Preferably, the subject of the pharmaceutical composition may be a mammal such as humans, canines, cats or rodents.

Preferably, the pharmaceutical composition may be administered to the skin surface by smearing.

Preferably, the frequency of administration of the pharmaceutical composition to a human subject may be from once every day to 3 times every day and the administration can be continued for at least 7 weeks.

According to the present invention, there is no specific limitation on the pharmaceutically acceptable excipient. For example, the pharmaceutically acceptable excipient may comprise water, propylene glycol of a combination thereof, but it is not limited thereto.

The present invention further provides a method for promoting hair growth, comprising administering to a subject in need thereof the pharmaceutical composition for promoting hair growth as described above.

The pharmaceutical composition for promoting hair growth in the method for promoting hair growth of the present invention is the same as that disclosed in the claimed object of the present invention.

In the method, the subject in need thereof may be a mammal such as humans, canines, cats or rodents.

In the method, the pharmaceutical composition may be administered to the subject in need thereof locally.

In the method, the pharmaceutical composition may be administered to the skin surface of the subject in need thereof by smearing.

The pharmaceutical composition as described above for use in promoting hair growth, comprising: administering to a subject in need thereof.

According to the present invention, the subject in need thereof may be a mammal.

According to the present invention, the pharmaceutical composition may be administered to the skin surface of the subject in need thereof by smearing.

According to the present invention, the pharmaceutical composition may be administered to the subject in need thereof locally.

The composition for promoting hair growth of the present invention can effectively promote hair growth in nude mice so that it can have effects of high hair coverage and promoting the increase of new hair follicles.

### IN THE DRAWINGS:

FIG. 1 shows the average hair coverage on the dressed skin of mice respectively after smeared with Example 1 and Comparative Examples 1 to 4 at 1^{st} to 7^{th} weeks after treatment.
FIGs. 2A to 2H are the photos taken by the optical microscope, showing the hair follicles of mice smeared with Example 1 and Comparative Examples 1 to 4 at the 7^{th} week; wherein the scale bar therein is 100 µm.

The present invention will now be further illustrated by the following examples.

### Reagent Description

1. An aqueous solution of silver nanoparticles: Product model: DA04-21; CAS No. 7440-22-4; purchased from LIWEI Nano Tech Co., Ltd.; the concentration of silver nanoparticles being from 0.00085 wt% to 0.0015 wt%; water being in an amount of from 99.9985wt % to 99.99915 wt%.
2. PVP: Product model: SI-PVP40-500G; CAS No. 9003-39-8; purchased from UNI-ONWARD Corp.
3. PMT extract liquid: Product model: 300558; purchased from GREENTECH BIOTECHNOLOGIES; the proportions of composition thereof: water: 49.0 wt% to 49.5 wt%, propylene glycol: 49.0 wt% to 49.5 wt%, the extract of the roots of PMT: 1.0 wt% to 2.0 wt%.
4. Copper Tripeptide-1: Product model: 1310CSB00010050; CAS No. 49557-75-7; purchased from Taiwan High & Better Corp.

### Preparation Example 1: Preparation of a mixture comprising an extract of PMT and silver nanoparticles

100 mL of the aqueous solution of silver nanoparticles (LIWEI Nano Tech Co., Ltd., DA04-21, concentration of silver nanoparticles: from 0.00085 wt% to 0.0015 wt%) was poured into a beaker with handle and stirred with magnets; wherein the particle size of the silver nanoparticles was smaller than 10 nm. 2.5 g of PVP was slowly added into the aqueous solution of silver nanoparticles in batches, mixed and stirred until a completion of said addition, and then continuously stirred until completely dissolved and the resulting mixture became clear.

In addition, after the PMT extract liquid (GREENTECH BIOTECHNOLOGIES, 305558, concentration of the extract of the PMT: from 1 wt% to 2 wt%) was filtered through a 0.45 µm filter cup, 10 mL of the filtered PMT extract liquid was taken to add into the aforementioned mixture, to obtain a mixture comprising an extract of *Polygonum Multiflorum* Thunb and silver nanoparticles. Wherein the weight/volume ratio of the silver nanoparticles to the mixture comprising the extract of *Polygonum Multiflorum* Thunb and the silver nanoparticles was from 0.0077 mg/mL to 0.014 mg/mL; the weight/volume ratio of the extract of *Polygonum Multiflorum* Thunb to the mixture comprising the extract of *Polygonum Multiflorum* Thunb and the silver nanoparticles was from 0.91 mg/mL to 1.82 mg/mL; the concentration of the PVP was about 0.57 mM.

### Example 1: Composition for promoting hair growth

The mixture comprising the extract of *Polygonum Multiflorum* Thunb and the silver nanoparticles and an aqueous solution of Copper Tripeptide-1 with a concentration of 0.5 mg/mL were mixed, and their volume ratio was 2.5:1, so as to obtain the composition for promoting hair growth of this example.

In this example, 110 mL of the mixture comprising the extract of *Polygonum Multiflorum* Thunb and the silver nanoparticles and 44 mL of the aqueous solution of Copper Tripeptide-1 were used; wherein the concentration of the Copper Tripeptide-1 in the composition for promoting hair growth was 0.14 mg/mL.

### Comparative Examples 1 to 4: Composition for promoting hair growth

Comparative Examples 1 to 4 were similar to Example 1, except the volume ratio of the mixture comprising the extract of PMT and the AgNPs to the aqueous solution of Copper Tripeptide-1 with a concentration of 0.5 mg/mL. The volume ratio of the mixture comprising the extract of PMT and the AgNPs to the aqueous solution of Copper Tripeptide-1 in Comparative Examples 1 to 4 were 20:1, 10:1, 5:1 and 1:1, respectively. The compositions and the proportions thereof in each of Comparative Examples and Example were shown in Table 1 below.

**Table 1: Compositions of Example 1 and Comparative Examples 1 to 4**

| Group No. | Volume of the mixture comprising the extract of PMT and the AgNPs (mL) | Volume of the aqueous solution of Copper Tripeptide-1 (mL) | Volume ratio of the mixture comprising the extract of PMT and the AgNPs to the aqueous solution of Copper Tripeptide-1 | Weight/volume ratio of the Copper Tripeptide-1 to the composition for promoting hair growth (mg/mL) |
|---|---|---|---|---|
| Example 1 | 110 | 44 | 2.5:1 | 0.14 mg/mL |
| Comparative Example 1 | 110 | 5.5 | 20:1 | 0.02 mg/mL |
| Comparative Example 2 | 110 | 11 | 10:1 | 0.05 mg/mL |
| Comparative Example 3 | 110 | 22 | 5:1 | 0.08 mg/mL |
| Comparative Example 4 | 110 | 110 | 1:1 | 0.25 mg/mL |

### Test Example 1: Hair Coverage Experiment

Example 1 and Comparative Examples 1 to 4 were as samples to undergo the following experiments: Six-week-old nude mice (Nu/Nu mice) were kept for one to two weeks to allow adaptation, and then they were randomly divided into groups, 5 mice for each group. A silicone splint (Grace Bio-Labs, Bend, OR, USA, Product model: SKU: 476687) was sewn on the back of each mouse. The outer diameter, the inner diameter and the thickness of the silicone splint were respectively 24 millimeters (mm), 20 mm and 0.5 mm. The compositions for promoting hair growth of different Example, Comparative Examples or control groups were respectively administered to the mouse skin in the silicone splint on the back of the mice every day for a total of 7 weeks, with a maximum volume of 40 microliters (µL).

In this test example, mice administered with the mixture comprising the extract of PMT and the AgNPs (absent of cluding Copper Tripeptide-1) of Preparation Example 1 on the backs were taken as the positive control group; the mice not given any drug on their backs were taken as the negative control group. In addition, mice administered with 0.5 mg/mL of the aqueous solution of Copper Tripeptide-1 on the backs were taken as the Copper Tripeptide-1 control group. Mice were weighed once a week to confirm their health status. The hair growth patterns of the mice were photographed on days 0, 7, 14, 21, 28, 35, and 42 after the beginning of the experiment, that is, at 1^{st}, 2^{nd}, 3^{rd}, 4^{th}, 5^{th}, 6^{th}, and 7^{th} weeks.

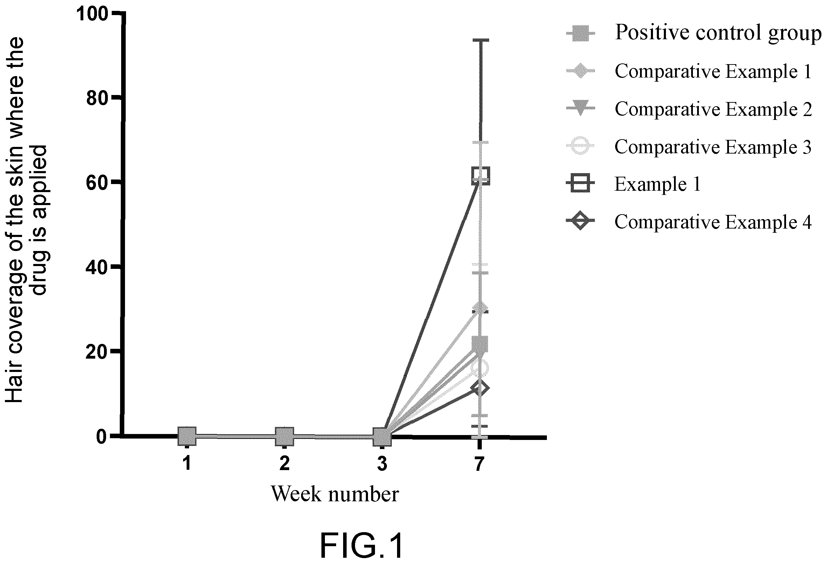

After the mice were anesthetized with 2.5% isoflurane, they were placed under a substantial microscope camera system (SZN71 Microscope system, Sunny, Hangzhou, China) to take photos by the built-in camera software; then, Image J software was used to analyze the hair coverage; the area of the inner diameter of the silicone splint attached to the backs of the mice (*i.e.* the area where the drug was administered) was selected and the total drug administration area was calculated; next, the area of the hair growth was selected and the area of the hair growth was calculated. The formula of hair coverage is: (the area of the hair growth ÷ the total drug administration area) x 100%, and the results of hair coverage of Example 1, Comparative Examples 1 to 4, and the control groups were shown in FIG. 1.

According to the average results of hair coverages of all mice in each group in FIG. 1, it can be seen that there was no obvious hair coverage in the area of the drug administration in each group from 1^{st} week to 3^{rd} week. At 7^{th} week, compared with the hair coverage of 30.6% of Comparative Example 1, 19.8% of Comparative Example 2, 16.4% of Comparative Example 3, 11.8% of Comparative Example 4 and 22.0% of the positive control group, respectively, the hair coverage of Example 1 was 61.8%, which was the best result. In addition, the results of hair coverage of each group at 7^{th} week were further analyzed by Two-way ANOVA (Tukey test) to determine whether there is significant difference in each group compared with the positive control group, and the results thereof were shown in Table 2 below. According to the results in Table 2, at 7^{th} week, compared to those of Comparative Examples 1 to 4, only the hair coverage of Example 1 was significantly different from the positive control group.

**Table 2: Example 1, Comparative Examples 1 to 4 with or without significant difference in each group compared with the positive control group**

| Group No. | With/without significant difference |
|---|---|
| Comparative Example 1 | No significant difference |
| Comparative Example 2 | No significant difference |
| Comparative Example 3 | No significant difference |
| Example 1 | *With significant difference |
| Comparative Example 4 | No significant difference |

### Test Example 2: Hair Follicles Count

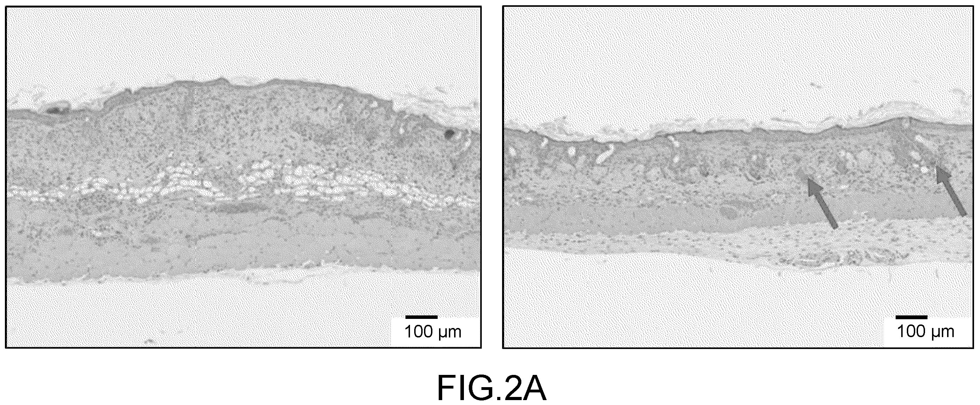

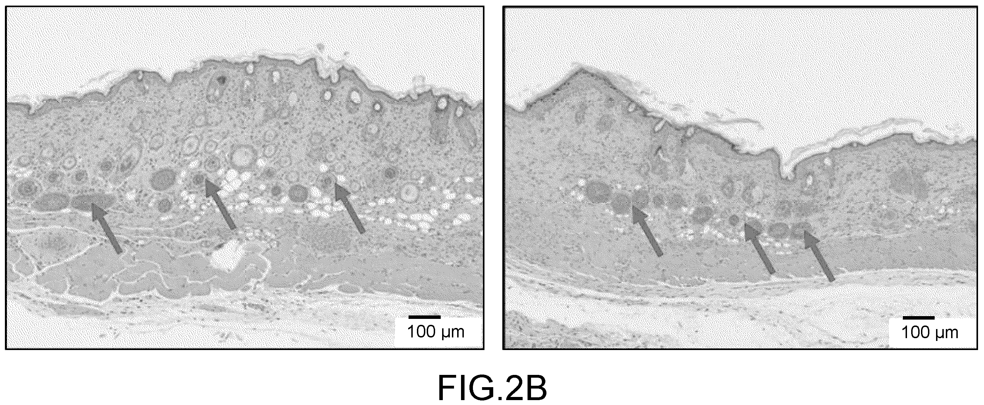

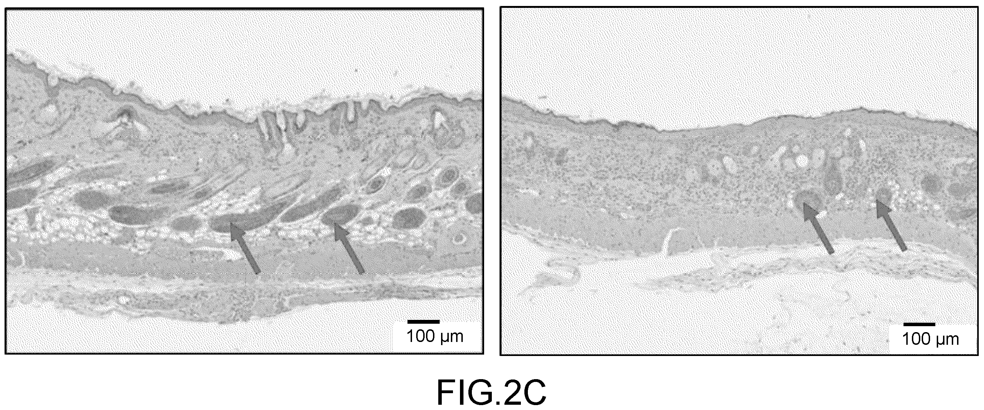

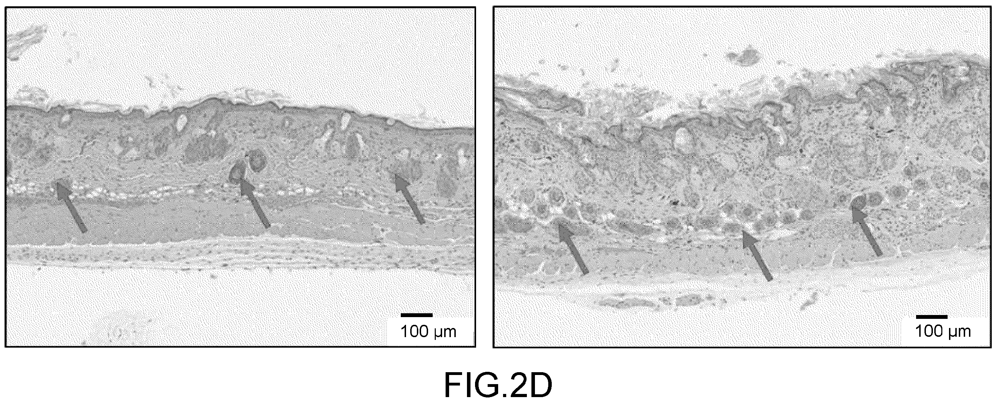

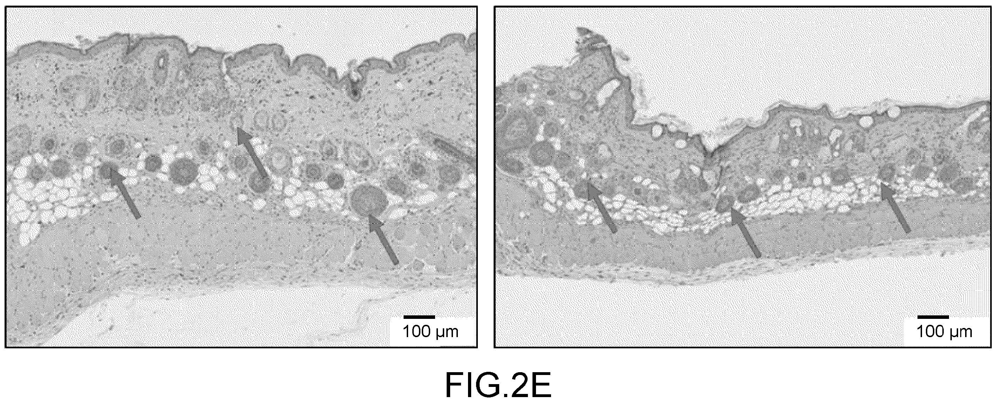

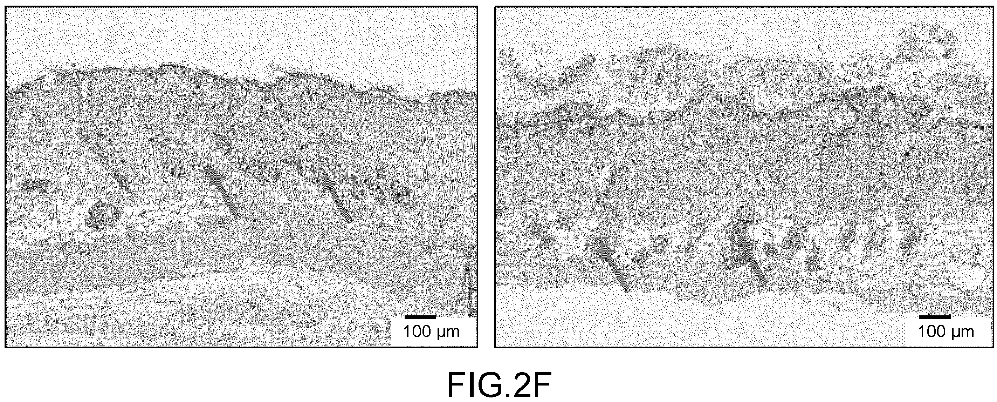

After sacrificing all mice in Test Example 1, mouse skin tissues were embedded in paraffin and then were cut into 3 µm-thick sections on a slide for Hematoxylin and eosin staining (H&E) and immunohistochemistry (IHC) staining for histological analysis. After H&E staining was performed, the stained slides were scanned by a slide panoramic scanner (OLYMPUS/VS200); with 80X magnification, two fields of each section were randomly selected for follicle observation and count, and the results were shown in FIGs. 2A to 2H.

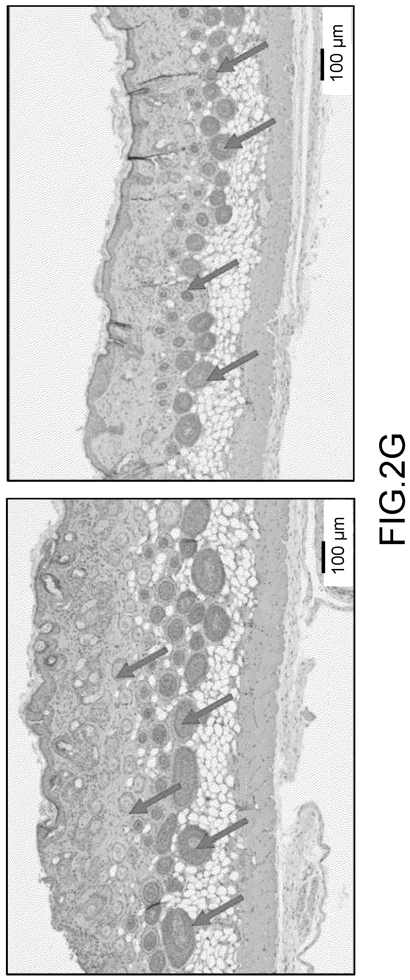

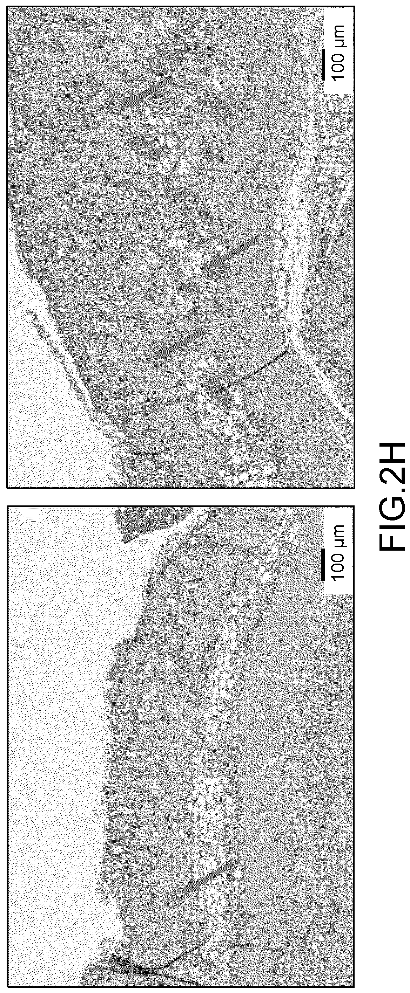

FIGs. 2A to 2H respectively were the sections of the negative control group, the positive control group, the Copper Tripeptide-1 control group, Comparative Example 1, Comparative Example 2, Comparative Example 3, Example 1, and Comparative Example 4, and the arrows on the images of each group referred to the exemplified quasi-circular-shaped hair follicles. Among them, as shown in FIG. 2G, the number of hair follicles in the section of the group treated with Example 1 was significantly higher than the number of hair follicles in the other groups (FIGs. 2A to 2F, and FIG. 2H), and the distribution of the hair follicles was denser. Therefore, the composition for promoting hair growth of the present invention can effectively promote the growth of new hair follicles.

In summary, the composition for promoting hair growth of the present invention can effectively promote hair growth in nude mice, and it can achieve the effects of high hair coverage and promoting the growth of new hair follicles.

## Claims

1. A composition for promoting hair growth, **characterized in that** the composition for promoting hair growth comprises:
a mixture comprising an extract of *Polygonum Multiflorum* Thunb and silver nanoparticles; and Copper Tripeptide-1;
wherein a weight/volume ratio of the Copper Tripeptide-1 to the composition for promoting hair growth is from 0.1 milligrams/milliliter (mg/mL) to 0.2 mg/mL;
wherein the mixture comprising the extract of *Polygonum Multiflorum* Thunb and the silver nanoparticles comprises the silver nanoparticles, the extract of *Polygonum Multiflorum* Thunb, a stabilizer, and a diluent.

2. The composition for promoting hair growth as claimed in claim 1, wherein a weight/volume ratio of the silver nanoparticles to the mixture comprising the extract *of Polygonum Multiflorum* Thunb and the silver nanoparticles is from 0.001 mg/mL to 0.1 mg/mL.

3. The composition for promoting hair growth as claimed in claim 1 or 2, wherein a particle size of the silver nanoparticles is larger than or equal to 1 nanometer (nm) and equal to or smaller than 50 nm.

4. The composition for promoting hair growth as claimed in any one of claims 1 to 3, wherein a weight/volume ratio of the extract of *Polygonum Multiflorum* Thunb to the mixture comprising the extract of *Polygonum Multiflorum* Thunb and the silver nanoparticles is from 0.1 mg/mL to 10 mg/mL.

5. The composition for promoting hair growth as claimed in any one of claims 1 to 4, wherein the extract of *Polygonum Multiflorum* Thunb is an extract of roots of *Polygonum Multiflorum* Thunb.

6. The composition for promoting hair growth as claimed in any one of claims 1 to 5, wherein the stabilizer comprises a protein, a peptide, sodium borohydride or polyvinyl pyrrolidone.

7. The composition for promoting hair growth as claimed in any one of claims 1 to 6, wherein the diluent is selected from the group consisting of: water, ethanol, dimethyl sulfoxide, acetonitrile, Hepes buffer, a phosphate buffer, Tris buffer, a citrate buffer, serum and any combinations thereof.

8. A pharmaceutical composition for promoting hair growth, **characterized in that** the pharmaceutical composition for promoting hair growth comprises: the composition for promoting hair growth as claimed in any one of claims 1 to 7; and a pharmaceutically acceptable excipient.

9. The pharmaceutical composition for promoting hair growth as claimed in claim 8, wherein the pharmaceutical composition is administered to a mammal as a subject in need thereof.

10. The pharmaceutical composition for promoting hair growth as claimed in claim 9, wherein the pharmaceutical composition is administered to the skin surface by smearing.

11. The pharmaceutical composition as claimed in claim 8 for use in promoting hair growth, comprising: administering to a subject in need thereof.

12. The pharmaceutical composition for use in promoting hair growth as claimed in claim 11, wherein the subject in need thereof is a mammal.

13. The pharmaceutical composition for use in promoting hair growth as claimed in claim 11 or 12, wherein the pharmaceutical composition is administered to the skin surface of the subject in need thereof by smearing.

14. The pharmaceutical composition for use in promoting hair growth as claimed in any one of claims 11 to 13, wherein the pharmaceutical composition is administered to the subject in need thereof locally.
